(19) Europäisches Patentamt
European Patent Office
Office européen des brevets

(11) **EP 4 477 213 A1**

(12) **EUROPEAN PATENT APPLICATION**
published in accordance with Art. 153(4) EPC

(43) Date of publication:
**18.12.2024 Bulletin 2024/51**

(21) Application number: **23752447.5**

(22) Date of filing: **10.02.2023**

(51) International Patent Classification (IPC):
*A61K 8/73* (2006.01)          *A61K 8/85* (2006.01)
*A61K 8/04* (2006.01)          *A61K 8/02* (2006.01)
*A61Q 19/08* (2006.01)

(86) International application number:
**PCT/CN2023/075506**

(87) International publication number:
**WO 2023/151669 (17.08.2023 Gazette 2023/33)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **10.02.2022   CN 202210125112**

(71) Applicant: **Sinclair Pharmaceuticals Limited
Chester CH4 9QT (GB)**

(72) Inventors:
• **ZHANG, Xuan
Hangzhou, Zhejiang 310011 (CN)**

• **WANG, Bin
Hangzhou, Zhejiang 310011 (CN)**
• **ZHANG, Hairu
Hangzhou, Zhejiang 310011 (CN)**
• **WANG, Zhuo
Hangzhou, Zhejiang 310011 (CN)**
• **XIA, Wenrong
Hangzhou, Zhejiang 310011 (CN)**
• **GAO, Jian
Hangzhou, Zhejiang 310011 (CN)**
• **DONG, Xijian
Hangzhou, Zhejiang 310011 (CN)**

(74) Representative: **Nederlandsch Octrooibureau
P.O. Box 29720
2502 LS The Hague (NL)**

(54) **INJECTABLE COSMETIC PRODUCT, AND PREPARATION METHOD THEREFOR AND USE THEREOF**

(57)     An injectable cosmetic product. The injectable cosmetic product comprises degradable microspheres and a CMC-Na gel, wherein the content of CMC-Na in the cosmetic product is 2-3 wt.%, the viscosity average molecular weight of CMC-Na in the CMC-Na gel is 0.8-1.2 million Da, and the shear viscosity of the CMC-Na gel is in the range of 40000-65000 mPa·s. Further provided are a method for preparing the injectable cosmetic product, and the use of the CMC-Na gel in the preparation of the injectable cosmetic product. By means of a CMC-Na carrier gel, microspheres can be uniformly suspended in the CMC-Na gel for a long time, and the respectively independent three-dimensional structures of the microspheres are retained, such that the microspheres can be uniformly distributed in a human body after injection, and the probability of lumps and foreign body granuloma is greatly reduced.

EP 4 477 213 A1

## Description

## Technical Field

The present invention relates to the field of medical cosmetology or medical preparations, and specifically to an injectable beauty product and a preparation method therefor and a use thereof.

## Background Art

Carboxymethyl cellulose (CMC) is a water-soluble cellulose ether obtained by the chemical modification of natural cellulose. Owing to the poor water-solubility of the acid structure of carboxymethyl cellulose, in order to better apply it, its products are generally made into sodium salt with the molecular formula $[C_6H_7O_2(OH)_2OCH_2COONa]n$. CMC-Na is a white fibrous or granular powder, and is odorless, tasteless, hygroscopic, and easy to disperse in water to form a transparent colloidal solution. CMC-Na has polymers with a wide range of molecular weights, degrees of substitution and degrees of polymerization. The gel formed by it has different properties, and has the advantages of being able to be stored for a long time without spoiling, high viscosity, strong shape retention, good biocompatibility and film-forming properties, etc. It has broad application prospects in food, daily necessities, medicine, etc.

Sodium carboxymethyl cellulose has many hydroxyl groups and carboxyl groups, is hygroscopic and can absorb a large amount of water. A hydrogel prepared using sodium carboxymethyl cellulose has a high water content, good hydrophilicity, a high swelling rate, good biocompatibility and biodegradability, and is widely used in the field of biomedicine. CMC-Na gel has the same rheological properties as many water-soluble macromolecular polymers. Low-molecular-weight CMC-Na gel is a flowable sol with low viscosity and tends to be fluid. Medium- and high-molecular weight CMC-Na gel has a higher viscosity and is a semisolid pseudoplastic fluid. In addition, CMC-Na gel is also thixotropic, that is, when the gel is subjected to an external force, its gel state tends to change to fluid, and when a mechanical force is removed, it will return to the previous gel state. Good thixotropy makes CMC-Na gel highly suitable for use as an injectable carrier material. Therefore, CMC-Na gel is often used as an injectable beauty product in the field of medical cosmetology.

Many of the injectable beauty products currently on the market use CMC-Na gel with low shear viscosity, which has low viscosity and poor thixotropy. Although it has good flowability and is easy to prepare, when CMC-Na gel with low viscosity and low thixotropy is used as a carrier for microspheres, micron-level particles will aggregate in the product, which is more obvious during the shelf life and storage of the product. It is also described in Patent Application CN112999990A that the improvement of microsphere suspension performance can reduce the adhesion and aggregation of microspheres during long-term storage. For degradable microspheres, after the number of microspheres is continuously degraded in the body, its concentration changes and it settles easily. Therefore, a technical problem that needs to be urgently solved in the prior art is how to obtain a degradable microsphere that is uniformly dispersed in CMC-Na gel and has better thixotropy, so that the filled microspheres can retain their uniform distribution in CMC-Na gel without aggregating, and the force and speed when a syringe is pushed are uniform and stable while avoiding needle blockage.

## Summary of the Invention

The present invention relates to an injectable beauty product, comprising degradable microspheres and CMC-Na gel. A CMC-Na carrier gel allows the microspheres to be uniformly suspended in the CMC-Na gel for a long time, maintaining the respectively independent three-dimensional structure of the microspheres, so that the microspheres are uniformly distributed in a human body after injection, greatly reducing the probability of lumps and foreign body granulomas. It was discovered through research that the dispersion degree of microspheres in CMC-Na gels of different properties is significantly different. CMC-Na gels of specific properties or specifications can effectively allow microspheres to be uniformly dispersed in the gel. Also, when the microspheres and CMC-Na gel are placed in a syringe for injection, the pushing force is moderate, the injection is uniform, and it is not easy to cause syringe blockage. Through research, the present invention has obtained a gel comprising degradable microspheres and CMC-Na, wherein the content of CMC-Na is 2 wt.% to 3 wt.%, the viscosity-average molecular weight is 800,000 to 1,200,000 Da, and the shear viscosity is in the range of 40,000 to 65,000 mPa•s. The product does not easily cause the deposition and aggregation of microspheres and the clogging of syringes, and has stable quality and good use effect throughout its shelf life, which greatly reduces adverse reactions.

An injectable beauty product, characterized by comprising degradable microspheres and CMC-Na gel, wherein the content of CMC-Na in the beauty product is 2 wt.% to 3 wt.%, the shear viscosity of the CMC-Na gel is in the range of 10,000 to 80,000 mPa•s, preferably in the range of 30,000 to 70,000 mPa•s, 40,000 to 65,000 mPa•s, or 30,000 to 50,000 mPa•s; the viscosity-average molecular weight of CMC-Na is 800,000 to 1,200,000 Da, and the degree of substitution (DS) is in the range of 0.7 to 1.0, preferably, 0.8 to 1.0 or 0.8 to 0.95. The viscosity-average molecular weight range may also be specifically and preferably selected from 900,000 Da, 950,000 Da, 1.1 million Da, and 1.15 million Da to obtain a better balance between the immediate filling effect and the sustained release effect.

In the injectable beauty product as described previously, the degradable microspheres are PCL microspheres, PLLA microspheres or PLGA microspheres.

In the injectable beauty product as described previously, the degradable microspheres have an average particle size in the range of 25 to 50 $\mu$m.

The injectable beauty product as described previously further comprises glycerol and a PBS buffer.

The injectable beauty product as described previously further comprises a syringe and a needle, wherein the degradable microspheres and the CMC-Na gel are pre-filled in the syringe.

A method for preparing the aforementioned CMC-Na gel, comprising: step 1: pouring a PBS buffer with a pH of 6.5 to 8 and prepared using dihydrogen phosphate and sodium hydroxide into a beaker, and placing the beaker on a stirring table to stir the buffer; step 2, slowly pouring CMC-Na so that the CMC-Na is fully in contact with the buffer, and stirring for 2 to 5 h until there are no lumps of CMC-Na in the gel, that is, there is no unstirred CMC-Na; step 3, adding glycerol to the gel, and stirring well for about 5 min; and step 4, using a vacuum stirring and degassing machine to carry out a stirring and degassing treatment.

A method for preparing the aforementioned injectable beauty product, comprising: step 1): pouring a PBS buffer with a pH of 6.5 to 8 and prepared using dihydrogen phosphate and sodium hydroxide into a beaker, and placing the beaker on a stirring table to stir the buffer; step 2), slowly pouring CMC-Na, which accounts for 2 wt.% to 3 wt.% of a beauty product filler, so that the CMC-Na is fully in contact with the buffer, and stirring for 2 to 5 h until there are no lumps of CMC-Na in the gel, that is, there is no unstirred CMC-Na; step 3), adding glycerol, which accounts for 0.9 wt.% to 1.1 wt.% of the beauty product filler to the gel, and stirring well for about 5 min; step 4), using a vacuum stirring and degassing machine to carry out a stirring and degassing treatment; and step 5), adding weighed 28 wt.% to 38 wt.% of microspheres to the gel, stirring preliminarily, and then using the vacuum stirring and degassing machine to carry out a stirring and degassing treatment.

Beneficial technical effects:

By detecting the properties (shear viscosity, thixotropy, etc.) of degradable microspheres in various CMC-Na gels, and combining the dispersion of microspheres in CMC-Na gels and the pushing force and pushing speed of injectable beauty products, it is finally determined that CMC-Na gels with a viscosity-average molecular weight of 800,000 to 1,200,000 Da and a shear viscosity of 40,000 to 65,000 mPa•s are the most suitable for injectable beauty products. Microspheres can be uniformly dispersed in the CMC-Na gels with said properties (whether before or after injection), effectively reducing the generation of lumps and foreign body granulomas; and when loaded into a syringe and pushed, the pushing force and pushing speed are moderate, making it easier for doctors to control the injection speed, improving injection accuracy, and making the push smoother and filling more uniform.

## Brief Description of the Drawings

FIGS. 1a and 1b are scanning electron microscope photographs of PCL microspheres with Mw=10,000 Da and 40,000 Da, respectively;

FIG. 2 is a scanning electron microscope photograph of PLGA microspheres;

FIG. 3 is a scanning electron microscope photograph of PLLA microspheres;

FIGS. 4a, 4b, 4c, 4d, 4e and 4f are microscopic images of 6 types of CMC-Na gels loaded with PCL microspheres; and FIGS. 4e' and 4f are microscopic images of CMC-Na gels Nos. 5 and 6 loaded with PCL microspheres after being squeezed out of a syringe.

## Detailed Description of the Preferred Embodiments

The present invention will be further described by examples below, but is not limited thereto.

**Example 1:** Preparation of PCL microspheres

Preparation method: 500 mg PCL was vortexed and dissolved in 2.5 ml DCM to form an oil phase; the oil phase was added dropwise to 25 ml of a 1% MC solution while stirring, then stirred for 30 s, sheared for 1 min at 14,000 r/min using a high-speed shear mixer, and stirred for 3 h at 1000 r/min; and it was washed with water and centrifuged 3 times, each time for 10 min, pre-frozen for 2 h, and dried for 24 h.

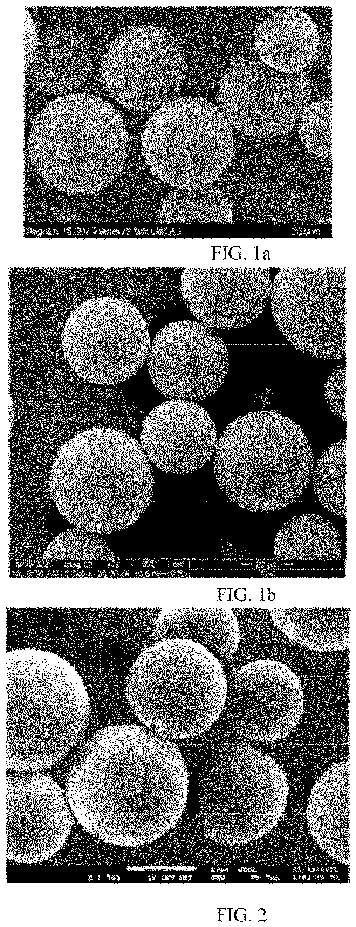

It was discovered by means of optical microscopic observation that the prepared microspheres had good roundness and appropriate particle size (see FIGS. 1a and 1b). The particle size range of the PCL microspheres was analyzed using a morphological particle size analyzer (Topsizer Plus), the process being as follows:

Weigh 0.1 g of microspheres and place them in a centrifuge tube; add 10 ml of water, oscillate, disperse, and ultrasonically treat for 30 min; then disperse them in a beaker pre-filled with 500 mL of deionized water; and use the OMEC particle size analyzer Topsizer Plus to measure the particle size, measure three times, and take an average value thereof. After detection and analysis, the particle size distribution of the PCL microspheres is relatively uniform, wherein 20 to 50 $\mu$m microspheres account for about 65%. Preferably, microspheres smaller than 20 $\mu$m account for ≤1%, microspheres of 20 to 25 $\mu$m account for ≤19%, microspheres of 25 to 50 $\mu$m account for ≥65%, and microspheres larger than 50 $\mu$m account for ≤15%. More preferably, microspheres smaller than 20 $\mu$m account for ≤1%, microspheres of 20 to 25 $\mu$m account for ≤15%, microspheres of 25 to 50 $\mu$m account for ≥70%, and microspheres larger than 50$\mu$m account for ≤14%.

**Example 2:** Preparation of PLGA microspheres

Preparation method: 500 mg PLGA was vortexed and dissolved in 2.5 ml DCM, and then a 500 $\mu$l internal aqueous phase was added; it was vortexed for 5 min, added dropwise to 10 ml of a 2% PVA solution while stirring, then stirred for 1 min, then added to 150 ml of a 0.2% PVA solution, stirred for 2 min, sheared for 30 s at a speed of 15,000 r/min using a high-speed shear mixer, and stirred at the rotational speed of 1000 r/min for 3 h; and it was washed with water and centrifuged 3 times, each time for 10 min, pre-frozen for 2 h, and dried for 24 h.

It can be seen from FIG. 2 that the PLGA microspheres were smooth and had good roundness and appropriate particle size. According to the particle size analysis of the microspheres, the particle size distribution of the PLGA microspheres is relatively uniform, wherein microspheres of 20 to 50 $\mu$m account for about 40%.

**Example 3:** Preparation of PLLA microspheres

Preparation method: 500 mg PLLA was vortexed and dissolved in 2.5 ml DCM, and added to 10 ml of a 2% PVA solution; it was sheared for 3 min at a rotational speed of 15,000 r/min using a high-speed shear mixer, then added to 150 ml of a 0.2% PVA solution, and stirred at 1000 r/min for 3 h; and it was washed with water and centrifuged 3 times, each time for 10 min, pre-frozen for 2 h, and dried for 24 h.

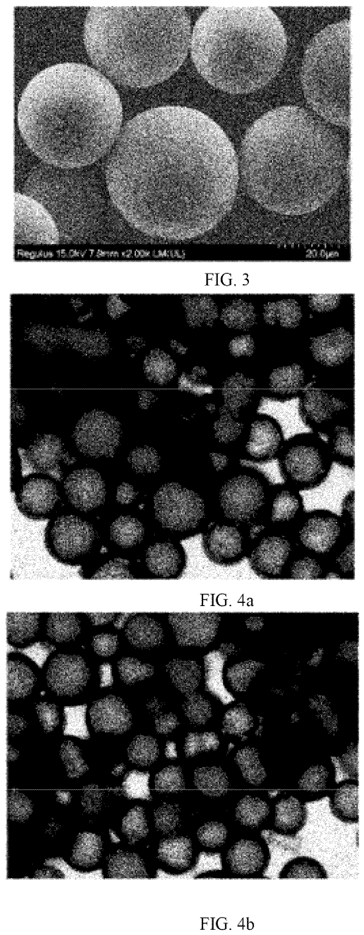

It can be seen from FIG. 3 that the PLLA microspheres were smooth, and had good roundness, good dispersibility, and appropriate particle size. The particle size distribution of the PLLA microspheres is relatively uniform, wherein microspheres of 20 to 50 $\mu$m account for about 32.18%.

**Example 4:** Detection of the viscosity-average molecular weight of 6 specifications of CMC-Na and preparation of CMC-Na gel.

Six different specifications of CMC-Na raw materials were selected from the market. The CMC-Na was purchased from Ashland LLC. and Sigma, respectively. The viscosity-average molecular weight of the above six specifications of CMC-Na was detected. The detection method was as follows:

Step 1: Accurately weigh 25 mg of a CMC-Na raw material, dissolve the raw material in about 40 mL of a 0.2 mol/L sodium chloride solution, and place the solution at 50°C for 16 h. Then, completely transfer the solution and fix the volume to 50 mL. Using an Ubbelohde viscometer with an inner diameter of 0.53 mm and a 0.2 mol/L sodium chloride solution as a blank control solution, determine the viscosity according to the General Rule 0633, Viscosity Determination Method (Method 2), of Part IV of the "Chinese Pharmacopoeia" (2020 Edition).

Step 2: Calculate the intrinsic viscosity [$\eta$] according to Formulas (1) and (2).

$$[\eta]= \ln\eta_r/c \qquad\qquad \text{Formula (1)}$$

$$\eta r = T/T0 \qquad\qquad \text{Formula (2)}$$

T is the outflow time of the test solution in s, T0 is the blank outflow time in s, and C is the concentration of the test solution;

Step 3: Calculate the viscosity-average molecular weight (M) according to Formula (3), i.e., the Mark-Houwink formula:

$$[\eta]=KM^\alpha \qquad\qquad \text{Formula (3)}$$

When sodium carboxymethyl cellulose was dissolved in a 0.2 mol/L sodium chloride solution, K=0.043 mL/g,

$\alpha$=0.74, and the viscosity-average molecular weights of the 6 specifications of CMC-Na described above were measured separately.

The degree of substitution and range of the degree of substitution of the CMC-Na gels are from the product label. The viscosity-average molecular weight and values of the degrees of substitution of the 6 specifications of CMC-Na gels are as shown in the following table:

Table 1

| No. | 1 | 2 | 3 | 4 | 5 | 6 |
|---|---|---|---|---|---|---|
| Viscosity-average molecular weight | 359781 | 568455 | 821654 | 1043366 | 1143576 | 1426951 |
| Degree of substitution | 0.79 | 0.73 | 0.91 | 0.88 | 0.78 | 0.80 |
| Range of degree of substitution | 0.65-0.90 | 0.65-0.90 | 0.86-0.95 | 0.80-0.95 | 0.65-0.90 | 0.70-0.95 |

Six specifications of CMC-Na raw materials were selected and respectively used for preparing gels. The preparation method was as follows:

step 1, pour a certain amount of PBS buffer with a pH of 6.5 to 8 and prepared using dihydrogen phosphate and sodium hydroxide into a beaker and stir;
step 2, slowly pour a certain amount of CMC-Na powder (accounting of 2 wt.% to 3 wt.% of a final product filler) so that the powder is fully in contact with the buffer, and stir for 2 to 5 h until there are no lumps in the gel, that is, there is no unstirred powder;
step 3, add a certain amount of glycerol (accounting of 0.9 wt.% to 1.1 wt.% of the final product filler) to the gel and stir well for about 5 min; and
step 4, use a vacuum stirring and degassing machine to carry out stirring and degassing treatment, and prepare 6 specifications of CMC-Na gels separately.

**Example 5:** Detection of shear viscosity and thixotropic index of 6 specifications of CMC-Na gels:
The methods for detecting thixotropic index and shear viscosity are as follows:
Take 10 to 20 ml of a gel sample, use a Brookfield LV type rotational viscometer, select the appropriate rotor, adjust the temperature to 25±1°C, and measure the dynamic viscosity at two rotational speeds of 5.6 r/min and 56 r/min, wherein the thixotropic index is the ratio of the dynamic viscosity at 5.6 r/min to that at 56 r/min.

Take 4.0 g of sodium carboxymethyl cellulose as a raw material, place it in a weighed 250 ml beaker, add 150 ml of hot water, keep it in a hot water bath for 30 min, and stir rapidly until the powder is completely wet. Cool, add enough water until the total weight of the mixture reaches 200 g, let it stand and stir from time to time until it is completely dissolved. Use a rotational rheometer (Brookfield DV2T rheometer), add an appropriate amount of a sample and place it on a test platform, and detect the shear viscosity under the conditions of 25°C and 1 s$^{-1}$ shear rate.

The shear viscosity and thixotropic index of the 6 specifications of CMC-Na gels are shown in Table 2:

Table 2

| | Shear viscosity/mPa•s | | | Thixotropic index | | |
|---|---|---|---|---|---|---|
| No. | No microspheres | Containing PCL microspheres Mw= 10,000 Da | Containing PCL microspheres Mw=40,000 Da | No microspheres | Containing PCL microspheres Mw= 10,000 Da | Containing PCL microspheres Mw=40,000 Da |
| 1 | 357.5 | 5481 | 6133 | 1.1 | 1.19 | 1.3 |
| 2 | 10080 | 20059 | 22104 | 2.03 | 2.68 | 2.73 |
| 3 | 40618 | 63759 | 60712 | 3.11 | 4.79 | 4.57 |
| 4 | 47606 | 81527 | 79823 | 4.56 | 4.22 | 4.05 |
| 5 | 64800 | 108716 | 98871 | 4.03 | 4.26 | 4.3 |
| 6 | 78121 | 134691 | 125143 | 5.74 | 5.39 | 5.1 |

**Example 6:** Mixing of PCL microspheres with CMC-Na gels

28 wt.% to 38 wt.% (which is the proportion in an injectable beauty product filler) of PCL microspheres which had been weighed were added to 6 CMC-Na gels which had been prepared, respectively; glycerol was added (wherein CMC-Na accounts for 2 wt.% to 3 wt.% of the injectable beauty product filler, and glycerol accounts for 0.9 wt.% to 1.1 wt.% of the injectable beauty product filler), and after preliminary stirring, a vacuum stirring and degassing machine was used to carry out a stirring and degassing treatment to mix uniformly; and the shear viscosity, thixotropic index, etc. after mixing were separately measured (see Table 2).

Table 2 shows the shear viscosity and thixotropic index of CMC-Na gels Nos. 1-6, and the shear viscosity and thixotropic index after adding PCL microspheres. It can be seen from Table 2 that the shear viscosity of CMC-Na gel No. 1 is much less than 10,000 mPa•s, the shear viscosity of CMC-Na gel No. 2 is around 10,000 mPa•s, and both of them have low shear viscosity. Moreover, the gel preparation process also shows that CMC-Na gels Nos. 1 and 2 have low shear viscosities, which are close to that of liquid fluid. The shear viscosities of CMC-Na gels Nos. 3 to 5 are in the range of 40,000 to 65,000 mPa•s, and the thixotropic indexes are between 3 and 5. They have moderate viscosities and certain fluid and colloid properties. The shear viscosity of CMC-Na gel No. 6 is above 65,000 mPa•s, up to 78,121 mPa•s.

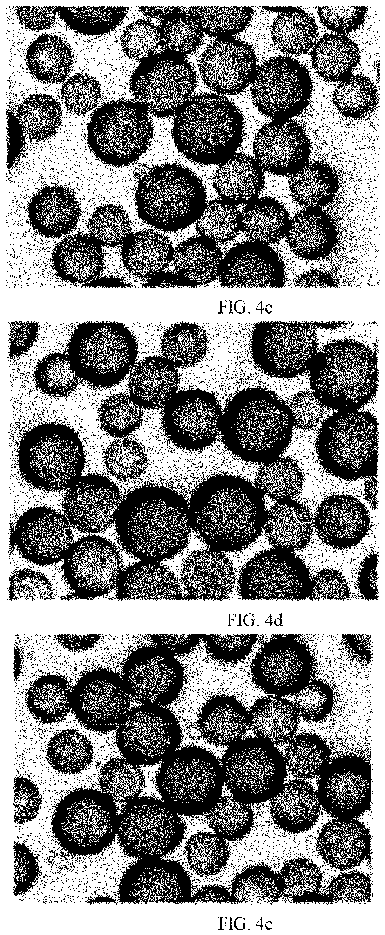

After adding PCL microspheres, both of the shear viscosity and thixotropic index of CMC-Na gels Nos. 1-6 changed, wherein the shear viscosity increased significantly and the thixotropic index also changed. There is little difference between PCL microspheres with molecular weights of 10,000 Da and 40,000 Da. After adding PCL microspheres with a weight-average molecular weight of 10,000 Da and 40,000 Da, the shear viscosity of gels Nos. 1 and 2 was about 5,500 and 20,000 mPa•s, respectively. The viscosity value was low, and the thixotropic index was less than 3. Compared with the gel to which no microspheres had been added, they were still close to fluid as a whole. After adding PCL microspheres, the shear viscosity of gels Nos. 3, 4 and 5 increased to 60,000 to 100,000 mPa•s, the thixotropic index was 3 to 5, and the overall state was gel-like, which was able to provide good support for microspheres (see FIGS. 4c to 4e). After adding microspheres to gel No. 6, the shear viscosity reached about 130,000 mPa•s, and the thixotropic index was greater than 5. During the preparation process, it could be clearly felt that the gel was viscous and difficult to stir.

For an injectable beauty product containing PCL microspheres, the choice of CMC-Na gel has a great impact on the product, because the degree of dispersion of PCL microspheres in the gel will directly affect the product quality, and the uniform dispersion of PCL microspheres will greatly reduce the probability of lumps and foreign body granulomas. It is known in the art that the degree of dispersion of PCL microspheres in CMC-Na gel is highly correlated with the shear viscosity of CMC-Na gel. Low-viscosity CMC-Na gel will cause the microspheres to settle and aggregate in the fluid gel (especially during long-term storage of the product), and CMC-Na gel with an overly high viscosity will make it difficult to mix the microspheres and cause the distribution thereof to be uneven. Therefore, it is crucial to select a CMC-Na gel with a certain shear viscosity for injectable beauty products. In addition, CMC-Na gel also has a certain thixotropy (which can be characterized by a thixotropic index), which makes CMC-Na gel suitable as an injectable carrier material. Because injectable beauty products require microspheres and CMC-Na gel to be loaded into a syringe, the thixotropy also needs to meet certain requirements to support the tendency of the gel to be a fluid when subjected to force, so that the injection is stable and controllable, and the gel can also quickly return to a viscous gel state after injection to maintain uniform suspension of the microspheres and reduce precipitation, aggregation and so on. This property of CMC-Na gel plays an important role in the stable injection of injectable beauty products.

**Example 7:** Microscope observation

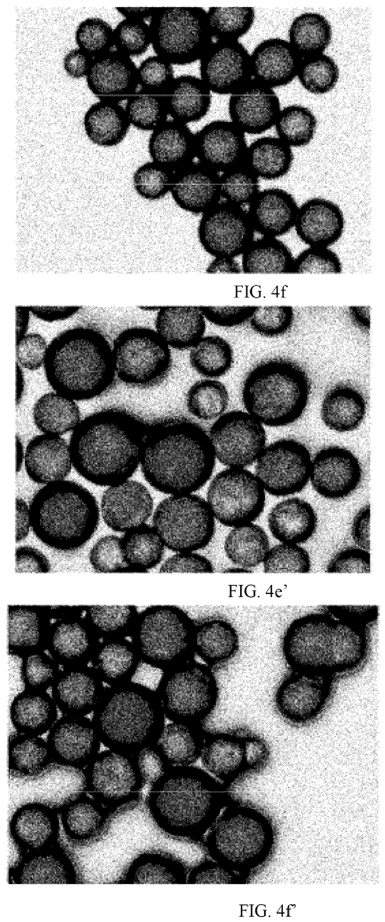

The PCL microspheres obtained in Example 6 (taking a weight-average molecular weight of 10,000 as an example) were stirred and mixed well in 6 specifications of CMC-Na gels, respectively, and observed under a microscope (XP-550C polarizing microscope) after standing. Microscopic photographs are shown in FIGS. 4a to 4f, which correspond to CMC-Na gels Nos. 1-6, respectively. It can be seen from FIGS. 4a and 4b that the microspheres in CMC-Na gels Nos. 1 (the viscosity-average molecular weight of which is 359,781, and the shear viscosity of which is 357.5) and 2 (the viscosity-average molecular weight of which is 568,455, and the shear viscosity of which is 10,080 mPa•s) all settled at the bottom and aggregated into piles, which may be related to the fact that CMC-Na gels Nos. 1 and 2 tend to be more fluid and intrinsically lack support for the microspheres. According to existing data, the aggregation of microspheres in injectable beauty products can easily lead to the formation of lumps and foreign body granulomas, and increase inflammatory response. It can be seen from FIGS. 4c, 4d and 4e that the microspheres in CMC-Na gels Nos. 3 (the viscosity-average molecular weight of which is 821,654, and the shear viscosity of which is 40,618), 4 (the viscosity-average molecular weight is 1,043,366, and the shear viscosity of which is 47,606), and 5 (the viscosity-average molecular weight of which is 1,143,576, and the shear viscosity of which is 64,800) can be uniformly dispersed, and there is less adhesion between the microspheres, and the uniformity is better. Since sufficient support can be provided, the microspheres will not settle in the gel. It can be seen from FIG. 4f that the microspheres in CMC-Na gel No. 6 (the viscosity-average molecular weight of which is 1,426,951, and the shear viscosity of which is 78,121) were unevenly dispersed, which was also obvious when stirring and mixing. The microspheres were easily scattered throughout the gel and aggregated with each other. Although mixing and stirring were carried out, it was still difficult to uniformly disperse the microspheres in the gel. This may be related to the high viscosity of gel No. 6 and the difficulty of dispersing the microspheres in the high-viscosity gel. In summary, it can be seen from the microscopic results that the CMC-Na gels of specifications 3-5 can better disperse the microspheres and cause same to be uniformly distributed, which is highly beneficial for reducing lumps and foreign body granulomas in injectable beauty products.

**Example 8:** Determination of pushing force in syringe

PCL microspheres and CMC-Na gel were loaded into a syringe, and the pushing force was measured using an HSV-1K electronic tensile testing machine. The determination method was as follows: after a test sample is balanced for 1 h at room temperature, a test distance is set to full scale, and the test is started at a specific test speed (20 mm/min, 30 mm/min and 50 mm/min), and the pushing force data is recorded. The above three test speeds were selected, among which the pushing speed of 30 mm/min is a more ideal speed. Approaching this pushing speed can not only make it easier for doctors to operate and make doctors less likely to make mistakes, but also ensure that the product is injected uniformly into the skin. Injecting too quickly or too slowly will affect the injection quality and the sensation of injection.

PCL microspheres with a weight-average molecular weight of 10,000 Da and 40,000 Da and 6 specifications of CMC-Na gels were uniformly mixed and loaded into a syringe with a 27G needle, respectively, to test their pushing force. In order to observe the dispersion of microspheres in the CMC-Na gels after pushing, microscopic photographs of some injectable beauty product fillers after squeezing were also taken. See FIGS. 4e' to 4f. The test results show that the pushing force of CMC-Na gels Nos. 1 and 2 is relatively small at the pushing speed of 30 mm/min, and the pushing force of gel No. 1 is even 20 N or below. It is easy for doctors to apply a pushing force of >25 N during actual injection, thereby speeding up the injection speed and affecting the injection quality. However, CMC-Na gels Nos. 3 to 5 can all achieve a pushing force in the range of 25 to 40 N at the pushing speed of 30 mm/min. This moderate pushing force makes it easier for doctors to operate. Maintaining this constant pushing speed can ensure that the product is injected uniformly into the skin, and enable doctors to control the injection speed more accurately, thereby improving doctors' injection accuracy, making the push injection smoother and the filling more uniform, and thus making the injection effect more natural. In addition, it can significantly reduce the pain during injection, improve the comfort of the injection, and reduce the swelling after injection. The pushing force of CMC-Na gel No. 6 at the pushing speed of 30 mm/min reaches 40 N or more, which obviously requires a greater pushing force than gels Nos. 1 to 5. The greater pushing force not only makes doctors feel tired, but also significantly affects the doctors' control over the injection speed, making the injection faster or slower, increasing the pain during injection, and causing a sharp decline in the quality of injection. Please see Tables 3 and 4 for details.

Table 3 Gel loaded with PCL microspheres (Mw=10,000 Da)

| PCL microspheres (Mw=10000) | 27G (unit: N) | | |
|---|---|---|---|
| CMC-Na No. | 20 mm/min | 30mm/min | 50 mm/min |
| 1 | 15.61 | 17.94 | 19.93 |
| 2 | 17.84 | 19.95 | 20.27 |
| 3 | 23.68 | 25.18 | 27.77 |
| 4 | 28.27 | 32.75 | 32.63 |
| 5 | 31.19 | 35.01 | 39.32 |
| 6 | 38.92 | 40.93 | 42.54 |

Table 4 Gels loaded with PCL microspheres (Mw=40,000Da)

| PCL microspheres (Mw=40000) | 27G (unit: N) | | |
|---|---|---|---|
| CMC-Na No. | 20 mm/min | 30mm/min | 50mm/min |
| 1 | 16.03 | 18.37 | 20.55 |
| 2 | 18.60 | 21.07 | 22.13 |
| 3 | 25.29 | 27.88 | 31.86 |
| 4 | 27.7 | 32.64 | 34.27 |
| 5 | 30.54 | 34.96 | 40.39 |
| 6 | 38.41 | 41.33 | 43.17 |

Through microscopic observation, the microspheres of gels Nos. 3 to 5 can still be uniformly dispersed after being pushed by the syringe (because the microscopic photographs of gels Nos. 3 to 5 are similar, only the microscopic photograph of gel No. 5 after pushing is selected, as shown in FIG. 4e'). It can be seen that gels Nos. 3 to 5 can effectively regain their colloidal properties after the force (pushing force) is removed and keep the microspheres uniformly dispersed within same, which may be related to their specific shear viscosity and thixotropic index. FIG. 4f shows the image of gel No. 6 after being pushed by the syringe. It can be seen that after the PCL microspheres were squeezed out of the syringe, they were obviously aggregated, and the microspheres were adhered to each other, with traces of squeezing.

In addition, in order to detect the effect of PLGA and PLLA microspheres in the CMC-Na gels, CMC-Na gels with PLGA and PLLA microspheres added thereto were also prepared in the experiments, and the same detection and analysis were carried out on same. Their experimental results are similar to those of the PCL microspheres, and will not be explicated here.

In summary, it can be seen from the above analysis that the injectable beauty products containing microspheres prepared by means of CMC-Na with a viscosity-average molecular weight of 800,000 to 1,200,000 Da, a degree of substitution of 0.7 to 1.0, and a shear viscosity range of 40,000 to 65,000 mPa·s have uniform distribution of microspheres and are not prone to the formation of lumps and foreign body granulomas; and after being loaded into the syringe, they can be injected into the skin with a constant pushing force and pushing speed, enabling the doctors to control the injection speed more accurately, filling more uniformly, and having a more natural injection effect. Meanwhile, it also significantly reduces the pain during injection and improves the comfort of injection.

Only the preferred embodiments of this patent are described above. It should be pointed out that for those of ordinary skill in the art, without departing from the technical principles of this patent, several improvements and replacements can also be made, and these improvements and replacements should also be regarded as the scope of protection of this patent.

## Claims

1. An injectable beauty product, **characterized by** comprising degradable microspheres and a CMC-Na gel, wherein the content of CMC-Na in the beauty product is 2 wt.% to 3 wt.%, the viscosity average molecular weight of the CMC-Na in the CMC-Na gel is 800,000 to 1,200,000 Da, and the shear viscosity of the CMC-Na gel is in the range of 40,000 to 65,000 mPa s.

2. The injectable beauty product as claimed in claim 1, **characterized in that** the degree of substitution of CMC-Na in the CMC-Na gel is in the range of 0.7 to 1.0.

3. The injectable beauty product as claimed in claim 1, **characterized in that** the injectable beauty product has a thixotropic index of 3 to 5.

4. The injectable beauty product as claimed in claim 1, **characterized in that** the degradable microspheres are PCL microspheres, PLLA microspheres or PLGA microspheres.

5. The injectable beauty product as claimed in claim 1, **characterized in that** the degradable microspheres have an average particle size in the range of 25 to 50 $\mu$m.

6. The injectable beauty product as claimed in claim 1, **characterized in that** the content of the degradable microspheres is 28% to 38%.

7. The injectable beauty product as claimed in claim 1, **characterized in that** it comprises glycerol and a PBS buffer.

8. The injectable beauty product as claimed in claim 1, **characterized in that** it further comprises a syringe in which the degradable microspheres and the CMC-Na gel are pre-filled.

9. The injectable beauty product as claimed in claim 1, **characterized in that** the preparation of the CMC-Na gel comprises:

   step 1), pouring a PBS buffer with a pH of 6.5 to 8 prepared by dihydrogen phosphate and sodium hydroxide into a beaker, and stirring evenly;
   step 2), slowly pouring the PBS buffer into CMC-Na so that the CMC-Na is fully in contact with the PBS buffer, and stirring for 2 to 5 h;

step 3), adding glycerol and stirring for about 5 min; and
step 4), using a vacuum stirring and degassing machine to carry out stirring and degassing treatment.

**10.** A method for preparing the injectable beauty product as claimed in claim 1, **characterized by**:

step 1): pouring a PBS buffer with a pH of 6.5 to 8 prepared by dihydrogen phosphate and sodium hydroxide into a beaker, and stirring evenly;
step 2): slowly pouring the PBS buffer into CMC-Na so that the CMC-Na is fully in contact with the PBS buffer, and stirring for 2 to 5 h until there is no unstirred CMC-Na;
step 3), adding glycerol and fully stirring for about 5 min;
step 4), using a vacuum stirring and degassing machine to carry out stirring and degassing treatment; and
step 5): adding the microspheres, stirring preliminarily, and then using the vacuum stirring and degassing machine to carry out stirring and degassing treatment.

**11.** The method as claimed in claim 10, **characterized in that** in step 5), the microspheres accounting for 28 wt.% to 38 wt.% of the beauty product are added to the gel.

**12.** The method as claimed in claim 10, **characterized in that**: in step 2), CMC-Na accounts for 2 wt.% to 3 wt.% of the beauty product.

**13.** The method as claimed in claim 10, **characterized in that**: in step 3), glycerol accounts for 0.9 wt.% to 1.1 wt.% of the beauty product.

**14.** Use of the CMC-Na gel as claimed in claim 1 in the preparation of an injectable beauty product, **characterized in that** the injectable beauty product comprises degradable microspheres and a CMC-Na gel.

FIG. 1a

FIG. 1b

FIG. 2

FIG. 3

FIG. 4a

FIG. 4b

FIG. 4c

FIG. 4d

FIG. 4e

FIG. 4f

FIG. 4e'

FIG. 4f'

**EP 4 477 213 A1**

## INTERNATIONAL SEARCH REPORT

| International application No. |
|---|
| **PCT/CN2023/075506** |

| A. | CLASSIFICATION OF SUBJECT MATTER |
|---|---|

A61K8/73(2006.01)i;A61K8/85(2006.01)i;A61K8/04(2006.01)i;A61K8/02(2006.01)i;A61Q19/08(2006.01)i

According to International Patent Classification (IPC) or to both national classification and IPC

| B. | FIELDS SEARCHED |
|---|---|

Minimum documentation searched (classification system followed by classification symbols)

A61K, A61Q

Documentation searched other than minimum documentation to the extent that such documents are included in the fields searched

Electronic data base consulted during the international search (name of data base and, where practicable, search terms used)

DWPI, WPABS, CNABS, CNTXT, EPTXT, USTXT, WOTXT, CNKI, 万方, WANFANG, ISI WEB OF SCIENCE, PubMed, Baidu: 辛克莱制药, 欣可丽美学, 中国远大, 张璇, 王斌, 张海茹, 王卓, 夏雯蓉, 高健, 董西健, 注射, 微球, 降解, 凝胶, 羧甲基纤维素, 羧甲基纤维素钠, PCL, PLLA, PLGA, 聚己内酯, microsphere, CMC-na, Carboxymethyl Cellulose, gel, inject

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 114146020 A (CHINA GRAND ENTERPRISES, INC.) 08 March 2022 (2022-03-08) claims 1-15 | 1-14 |
| A | WO 2019139381 A1 (G2GBIO, INC.) 18 July 2019 (2019-07-18) embodiment 6, and description, page 10, and paragraph 2 | 1-14 |
| A | CN 112999426 A (JIANGSU XIHONG BIOLOGY MEDICINE CO., LTD.) 22 June 2021 (2021-06-22) embodiment 1, and description, paragraphs 7 and 28 | 1-14 |
| A | CN 111298196 A (CHANGZHOU INSTITUTE OF MATERIA MEDICA CO., LTD.) 19 June 2020 (2020-06-19) embodiment 4, and description, paragraph 46 | 1-14 |
| A | WO 2019139380 A1 (G2GBIO, INC.) 18 July 2019 (2019-07-18) Embodiment 6 | 1-14 |
| A | WO 2021233967 A1 (INTERVET INTERNATIONAL B.V.) 25 November 2021 (2021-11-25) claims 1-4 and 6 | 1-14 |

☑ Further documents are listed in the continuation of Box C.   ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **20 April 2023** | **28 April 2023** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

14

**INTERNATIONAL SEARCH REPORT**

International application No.

**PCT/CN2023/075506**

**C.    DOCUMENTS CONSIDERED TO BE RELEVANT**

| Category* | Citation of document, with indication, where appropriate, of the relevant passages | Relevant to claim No. |
|---|---|---|
| A | CN 111886031 A (G2GBIO, INC.) 03 November 2020 (2020-11-03)<br>      entire document | 1-14 |
| A | CN 113694252 A (UNIVERSITY OF JINAN) 26 November 2021 (2021-11-26)<br>      entire document | 1-14 |
| A | WO 0191720 A2 (ALKERMES CONTROLLED THERAPEUTICS, INC.I) 06 December 2001 (2001-12-06)<br>      entire document | 1-14 |

Form PCT/ISA/210 (second sheet) (July 2022)

<table>
<tr><td colspan="3" align="center">**INTERNATIONAL SEARCH REPORT**<br>**Information on patent family members**</td><td colspan="2">International application No.<br>**PCT/CN2023/075506**</td></tr>
</table>

| Patent document<br>cited in search report | | | Publication date<br>(day/month/year) | Patent family member(s) | | | Publication date<br>(day/month/year) |
|---|---|---|---|---|---|---|---|
| CN | 114146020 | A | 08 March 2022 | None | | | |
| WO | 2019139381 | A1 | 18 July 2019 | None | | | |
| CN | 112999426 | A | 22 June 2021 | None | | | |
| CN | 111298196 | A | 19 June 2020 | None | | | |
| WO | 2019139380 | A1 | 18 July 2019 | BR | 112020014046 | A2 | 01 December 2020 |
| | | | | EP | 3738622 | A1 | 18 November 2020 |
| | | | | EP | 3738622 | A4 | 22 September 2021 |
| | | | | JP | 2021511300 | A | 06 May 2021 |
| | | | | KR | 20190085499 | A | 18 July 2019 |
| | | | | KR | 102034871 | B1 | 08 November 2019 |
| | | | | US | 2021052768 | A1 | 25 February 2021 |
| | | | | US | 11590258 | B2 | 28 February 2023 |
| | | | | JP | 2022087133 | A | 09 June 2022 |
| | | | | RU | 2751507 | C1 | 14 July 2021 |
| WO | 2021233967 | A1 | 25 November 2021 | AU | 2021277491 | A1 | 01 December 2022 |
| | | | | CO | 2022016630 | A2 | 29 November 2022 |
| | | | | BR | 112022022923 | A2 | 20 December 2022 |
| | | | | CA | 3182659 | A1 | 25 November 2021 |
| | | | | EP | 4153133 | A1 | 29 March 2023 |
| | | | | IL | 298259 | A | 01 January 2023 |
| CN | 111886031 | A | 03 November 2020 | EP | 3738621 | A1 | 18 November 2020 |
| | | | | EP | 3738621 | A4 | 13 October 2021 |
| | | | | JP | 2021511112 | A | 06 May 2021 |
| | | | | JP | 7054954 | B2 | 15 April 2022 |
| | | | | WO | 2019139381 | A1 | 18 July 2019 |
| | | | | BR | 112020014068 | A2 | 01 December 2020 |
| | | | | RU | 2749803 | C1 | 17 June 2021 |
| | | | | US | 2020353127 | A1 | 12 November 2020 |
| | | | | KR | 20190085500 | A | 18 July 2019 |
| | | | | KR | 102034872 | B1 | 21 October 2019 |
| CN | 113694252 | A | 26 November 2021 | None | | | |
| WO | 0191720 | A2 | 06 December 2001 | CY | 1117734 | T1 | 17 May 2017 |
| | | | | DK | 1283699 | T3 | 01 October 2007 |
| | | | | WO | 0191720 | A3 | 23 May 2002 |
| | | | | CY | 1107441 | T1 | 19 December 2012 |
| | | | | PL | 365195 | A1 | 27 December 2004 |
| | | | | PL | 204298 | B1 | 31 December 2009 |
| | | | | HK | 1054319 | A1 | 28 November 2003 |
| | | | | HK | 1056321 | A1 | 13 February 2004 |
| | | | | BG | 107288 | A | 30 June 2003 |
| | | | | BG | 66023 | B1 | 30 November 2010 |
| | | | | DE | 60128798 | D1 | 19 July 2007 |
| | | | | DE | 60128798 | T2 | 31 October 2007 |
| | | | | RU | 2002135641 | A | 20 July 2004 |
| | | | | AU | 5546301 | A | 11 December 2001 |
| | | | | EP | 1283699 | A2 | 19 February 2003 |
| | | | | EP | 1283699 | B1 | 06 June 2007 |
| | | | | ES | 2286118 | T3 | 01 December 2007 |
| | | | | DK | 2269577 | T3 | 27 June 2016 |

Form PCT/ISA/210 (patent family annex) (July 2022)

International application No.

**PCT/CN2023/075506**

| Patent document cited in search report | Publication date (day/month/year) | Patent family member(s) | | | Publication date (day/month/year) |
|---|---|---|---|---|---|
| | | NZ | 522335 | A | 29 August 2003 |
| | | US | 2004208938 | A1 | 21 October 2004 |
| | | US | 7371406 | B2 | 13 May 2008 |
| | | US | 2014193507 | A1 | 10 July 2014 |
| | | DK | 1754469 | T3 | 27 June 2016 |
| | | CY | 1117652 | T1 | 17 May 2017 |
| | | US | 2008044478 | A1 | 21 February 2008 |
| | | HU | 0302283 | A2 | 28 October 2003 |
| | | HU | 0302283 | A3 | 28 February 2007 |
| | | HU | 228587 | B1 | 29 April 2013 |
| | | SI | 2269577 | T1 | 30 September 2016 |
| | | JP | 2003534366 | A | 18 November 2003 |
| | | JP | 4502355 | B2 | 14 July 2010 |
| | | IL | 152767 | A0 | 24 June 2003 |
| | | IL | 152767 | A | 29 December 2008 |
| | | MXPA | 02011543 | A | 12 August 2004 |
| | | EP | 3095442 | A1 | 23 November 2016 |
| | | EP | 3095442 | B1 | 18 November 2020 |
| | | EP | 1754469 | A1 | 21 February 2007 |
| | | EP | 1754469 | B1 | 23 March 2016 |
| | | ES | 2572877 | T3 | 02 June 2016 |
| | | CZ | 20023848 | A3 | 14 May 2003 |
| | | CZ | 301359 | B6 | 27 January 2010 |
| | | PT | 1283699 | E | 22 June 2007 |
| | | KR | 20030020285 | A | 08 March 2003 |
| | | KR | 100810480 | B1 | 07 March 2008 |
| | | PT | 2269577 | E | 14 June 2016 |
| | | IS | 6595 | A | 28 October 2002 |
| | | IS | 2471 | B | 15 December 2008 |
| | | US | 2010303900 | A1 | 02 December 2010 |
| | | AU | 2001255463 | B2 | 04 November 2004 |
| | | ES | 2574823 | T3 | 22 June 2016 |
| | | US | 2003113380 | A1 | 19 June 2003 |
| | | US | 6667061 | B2 | 23 December 2003 |
| | | US | 6495164 | B1 | 17 December 2002 |
| | | AT | 363891 | T | 15 June 2007 |
| | | HK | 1152660 | A1 | 09 March 2012 |
| | | BR | 0111060 | A | 15 April 2003 |
| | | BRPI | 0111060 | B1 | 16 April 2019 |
| | | BRPI | 0111060 | B8 | 25 May 2021 |
| | | EP | 2269577 | A2 | 05 January 2011 |
| | | EP | 2269577 | A3 | 30 November 2011 |
| | | EP | 2269577 | B1 | 16 March 2016 |
| | | US | 2008044485 | A1 | 21 February 2008 |
| | | US | 7799345 | B2 | 21 September 2010 |
| | | SI | 1754469 | T1 | 30 September 2016 |
| | | NO | 20025164 | D0 | 28 October 2002 |
| | | NO | 20025164 | L | 25 November 2002 |
| | | NO | 334660 | B1 | 12 May 2014 |
| | | HK | 1103346 | A1 | 21 December 2007 |

Form PCT/ISA/210 (patent family annex) (July 2022)

**INTERNATIONAL SEARCH REPORT**
Information on patent family members

International application No.

**PCT/CN2023/075506**

| Patent document cited in search report | Publication date (day/month/year) | Patent family member(s) | | | Publication date (day/month/year) |
|---|---|---|---|---|---|
| | | CA | 2406536 | A1 | 06 December 2001 |
| | | CA | 2406536 | C | 22 December 2009 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 112999990 A

**Non-patent literature cited in the description**

- Chinese Pharmacopoeia. 2020